# EUROPEAN PATENT SPECIFICATION

(11) **EP 3 181 118 B1**
(45) Date of publication and mention of the grant of the patent: **13.02.2019**
(21) Application number: 15461585.0
(22) Date of filing: 14.12.2015
(51) Int. Cl.: A61K 9/00, A61K 31/375, A61K 31/66, A61K 31/7012, A61K 33/24, A61P 35/02

(54) **SYNERGISTIC COMBINATION OF TXNR INHIBITORS AND ASCORBATE FOR TREATMENT OF B CELL MALIGNANCIES**
SYNERGISTISCHE KOMBINATION AUS TXNR-INHIBITOREN UND ASCORBAT ZUR BEHANDLUNG VON B-ZELL-MALIGNOMEN
COMBINAISON SYNERGIQUE D'INHIBITEURS DE TXNR ET D'ASCORBATE POUR LE TRAITEMENT DE MALIGNITÉS DES CELLULES B

(43) Date of publication of application: 21.06.2017
(73) Proprietor: Warszawski Uniwersytet Medyczny, 02-091 Warszawa (PL)
(72) Inventor: ZAGOZDZON, Radoslaw, 05-080 Truskaw (PL); FIRCZUK, Malgorzata, 02-775 Warszawa (PL); GRACZYK-JARZYNKA, Agnieszka, 02-807 Warszawa (PL); TRZECIECKA, Anna, Miami, FL 33133 (US); BAJOR, Malgorzata, 02-237 Warszawa (PL)
(74) Representative: Patpol Kancelaria Patentowa Sp. z o.o.

(56) References cited:
- WO-A1-2014/124384
- WO-A1-2015/181737
- DAI J ET AL: "MALIGNANT CELLS CAN BE SENSITIZED TO UNDERGO GROWTH INHIBITION AND APOPTOSIS BY ARSENIC TRIOXIDE THROUGH MODULATION OF THE GLUTATHIONEREDOX SYSTEM", BLOOD, AMERICAN SOCIETY OF HEMATOLOGY, US, vol. 93, no. 1, 1 January 1999 (1999-01-01), pages 268-277, XP000891685, ISSN: 0006-4971

## Description

### TECHNICAL FIELD

The invention provides a prooxidant synergistic combination to treat B-cell malignancies, such as B cell lymphomas, which comprises a thioredoxin reductase (TXNR) inhibitor, auranofin (AUR), and L- or D- ascorbate (L-ASC or D-ASC). The combination of the invention exerts synergistic cytotoxic effects towards malignant, but not normal B cells.

### BACKGROUND ART

### B cell lymphomas

B cell lymphomas are the most common (86%) subtype of non-Hodgkin lymphomas (NHL) and are derived from the mature stage B lymphocytes. The incidence of this type of lymphoma varies depending on the geographic location. In Poland, the estimated incidence ranges between 10 and 18 new cases per 100 000 citizens per year. B cell lymphomas are placed in the top ten regarding the incidence and cancer-related mortality in adults. Therefore, they pose a significant social problem, especially since the occurrence of this type of cancer in the Polish population continues to grow. B cell NHL are classified depending on growth rate as indolent and aggressive. The most common aggressive B cell lymphomas are diffuse large B cell lymphoma (DLBCL), Burkitt lymphoma (BL) and mantle cell lymphoma. Lymphoma is mainly treated with chemotherapy. Prognosis and overall survival vary greatly, depending on the stage at diagnosis and type of the disease, and is worse for aggressive lymphomas. Approximately 10-30% of cases relapse as refractory, chemo-resistant disease. New therapeutic approaches are needed to better cure refractory disease and decrease side effects of the existing aggressive treatment [1].

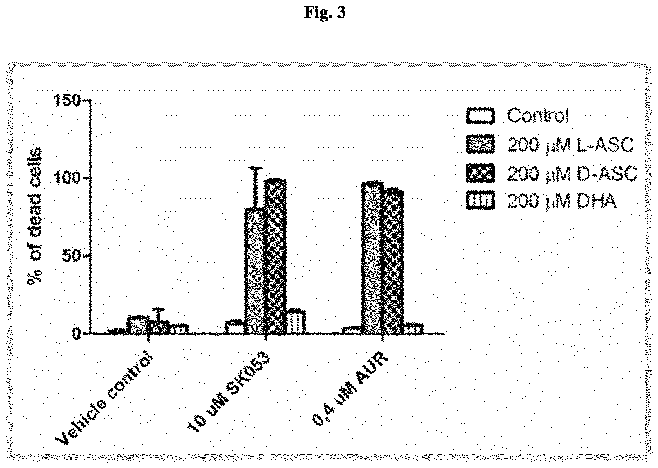

One of the classic characteristics of many types of cancers is the presence of elevated oxidative stress within cancer cells. Oxidative stress has been traditionally defined as an imbalance between reactive oxygen species (ROS) production and removal. The two major ROS scavenging machineries in cells are thioredoxin/thioredoxin reductase (TXN/TXNR) and glutathione (GSH) systems. Increasing data suggest that lymphoma cells are subject to higher levels of oxidative stress and therefore are particularly susceptible to ROS-inducing agents, some of them, such as imexon [2], are currently evaluated as anti-lymphoma therapeutics in clinical trials. As published by Chen et al. [3], (Fig.3), lymphoma cells are much more sensitive to direct exposure to exogenous H₂O₂ as compared to normal B cells. Accordingly, lymphoma cells are among the most sensitive to L-ASC (LD₅₀ ∼ 0,5 mM), which generates exogenous H₂O₂. However, despite the high sensitivity *in vitro,* the anti-lymphoma activity of high dose parenteral L-ASC *in vivo* is limited [4].

### Rationale for prooxidant therapies in lymphoma

Rapidly proliferating cells, such as aggressive B cell lymphomas, which are characterized by rapid growth rate, have increased levels of ROS [5]. It renders lymphoma cells particularly sensitive to ROS-inducing therapies and dependent on specific redox homeostasis enzymes belonging to two major cellular antioxidant systems: GSH and TXN/TXNR.

### TXN/TXNR system as a target in antitumor therapy

TXN/TXNR system consists of TXN, TXNR and NADPH. Humans express three TXNR isoforms: TXNR1 (cytosolic), TXNR2 (mitochondrial), TXNR3 (testis specific). TXNR1 is the only enzyme that reduces cytoplasmic TXN1, which in turn reduces disulfides of its target proteins, including transcription factors, ribonucleotide reductase, and also H₂O₂-scavenging enzymes - peroxiredoxins (PRDXs). Besides TXN, TXNR has a broad spectrum of substrates, ranging from small molecules such as selenite, lipid hydroperoxides, ebselen, and dehydroascorbate (DHA), to proteins, like protein disulfide isomerase or glutathione peroxidase. Most of these substrates are involved in cellular redox regulation, therefore, TXNR plays a central role in red-ox homeostasis. In tumors, TXN/TXNR system exhibits a wide range of functions including growth promoting, inhibiting apoptosis and increasing angiogenesis [6]. Numerous studies have demonstrated that TXN/TXNR system proteins are upregulated in many aggressive malignancies and their inhibition, especially TXNR as a superordinate enzyme of the system, can induce cell death or increase the tumor cell sensitivity to other therapeutic modalities [7, 8].

### TXNR inhibitors as antitumor agents

Numerous TXNR inhibitors have been investigated as anti-tumor drugs for recent decades, among them: metal-containing compounds - auranofin (AUR) and other gold complexes [9], motexafin gadolinium [10], arsenic trioxide [11], AW464 [12], as well as naturally occurring products e.g. curcumin [13]. An example of peptidomimetic inhibitor, SK053, aimed to target TXN was also disclosed [14]. The compound inhibits the activity of both TXN and TXNR, exerts cytostatic/cytotoxic effect with a clear preference towards rapidly proliferating cells, and has antitumor activity in mice with no evidence of systemic toxicity. In our further studies, in the cell line model of human Burkitt lymphoma, we have also identified dimeric PRDXs as additional targets for SK053 [15].

### Auranofin (AUR)

AUR is a (2,3,4,6-tetra-O-acetyl-1-thio-β-D-glucopyranosato-S-) (triethyl-phosphine) gold complex approved for the treatment of rheumatoid arthritis as a disease modifying agent. Since 1985, the only available preparation is Ridaura® - 3 mg orally administered capsules, each containing 0.87 mg of gold. The usual scheme of dosage in rheumatoid arthritis for adults is 6 mg daily given in 1 or 2 doses, that may be increased to 9 mg daily (Ridaura product monograph;[16]).

### AUR Pharmacokinetics

Pharmacokinetic studies with radiolabelled AUR in rheumatoid arthritis patients have demonstrated that approximately 25% of oral dose is being absorbed. Peak plasma concentrations of 0.3-0.4 µM are reached in 1-2 h. The mean plasma half-life was 17 days and by day 10, 77% of labelled gold was excreted - 73% with feces and 4% with urine. Six months after a single dose, only 0,4% of labelled gold was retained in the body. In blood approximately 40% of AUR gold is bound to red blood cells, while 60% to serum proteins ([16], Ridaura product monograph).

AUR overdosage data is limited - 50-year-old female developed toxicity after 10-day treatment with 27 mg of AUR daily but recovered after withdrawal. In animal models, acute oral LD₅₀ is 310 mg/kg in adult mice and 265 mg/kg in adult rats. The minimum lethal dose in rats is 30mg/kg (Ridaura product monograph).

### AUR Pharmacodynamics

The main mechanism of action of AUR is inhibition of red-ox enzymes via formation of irreversible adducts of AUR to thiol and selenol groups. The compound is particularly potent and specific inhibitor of selenoenzyme TXNR [17, 18]. Other putative mechanisms of AUR activity were also reported, such as inhibition of ubiquitin-proteasome system [19].

### Rediscovery of AUR as anti-cancer agent

Currently AUR is of special interest for its possible new therapeutic application in many diseases including neoplasms. Cytotoxicity of AUR was demonstrated in solid tumors cell lines: ovarian cancer, lung adenocarcinoma, gastric cancer as well as in hematological malignancies: T cell leukemia, monocytic leukemia, primary chronic lymphocytic leukemia (CLL) and acute myeloid leukemia (AML) cells, cell lines and primary mantle cell lymphoma cells, chronic myeloid leukemia (CML) expressing Bcr-Abl. Regarding B cell malignancies, Mirabelli et al.[20] have shown potent *in vivo* anti-tumour activity of AUR administered intraperitoneally (i.p.) (8 mg/kg daily) in P388 murine monocytic leukaemia model. Fiskus et al.[21] demonstrated AUR pro-apoptotic activity against CLL using MEC-1 cell line and primary CLL cells. Interestingly, AUR anti-leukemic activity was independent of classic mechanisms of chemotherapy resistance. Currently, AUR is being evaluated in the ongoing phase I/II clinical trial in CLL/SLL(small lymphocytic lymphoma)/PLL(prolymphocytic leukemia) patients - 6 mg of AUR administered orally, twice daily. Analysis of blood samples from 6 patients treated with AUR only (NCT01419691), revealed transiently decreased anti-oxidant defense markers, increase in cellular ROS levels and induction of apoptosis. The transient nature of the cellular response *in vivo* was consistent with the limited clinical activity of AUR treatment seen in patients (Saba, 2013 ASH Conference, abstract 3819). Celegato et al.[22] in classic Hodgkin lymphoma demonstrated anti-tumor activity of AUR alone and clear synergism in combinations with doxorubicin, cisplatin or gemcitabine. Additionally, in mantle cell lymphoma, AUR synergized with buthionine sulphoximine (BSO), a compound decreasing glutathione levels [23], and with PARP inhibitor ABT-888 [Gangulyn 2015 AACR 106th Annual Meeting].

### Ascorbate (ASC) as an essential nutrient and its transport to cells

L-ASC, widely recognized as vitamin C, is an essential nutrient for humans. L-ASC is a cofactor of several enzymes. It supports collagen synthesis and therefore its deficiency causes impairment of connective tissue. Some animals and plants are able to synthesize L-ASC, but humans lack the key L-ASC biosynthesis enzyme and require it as a part of their diet. ASC has two chiral centers and therefore occurs in the form of four stereoisomers. The enantiomer of L-ASC, D-ASC, also known as erythorbic acid, has identical redox properties, but limited activity as vitamin C, as it lacks antiscorbutic activity. D-ASC is widely used as a food preservative.

ASC has antioxidant, mild reducing properties. In a culture medium or an extracellular space, ASC is oxidized first to an ascorbate radical, and then with a loss of a second electron, to a fully oxidized form, dehydroascorbic acid (DHA). ASC and DHA enter cells using distinct protein transporters. DHA enters cells via glucose transporters, GLUT 1, 2, and 4, which are modestly stereo-selective to L-DHA. However, under physiological conditions this way of vitamin C uptake does not play a major role due to high concentration (5-10 mM) of competing glucose. Reduced L-ASC is transported to cells via Na⁺-dependent protein carriers, SVCT1 and SVCT2, which are selective for L-ASC. Following cell entry, DHA is reduced back to ASC at the expense of GSH and TXN/TXNR systems [24].

### Ascorbate (ASC) as anticancer agent - preclinical studies

Many *in vitro* studies revealed that L-ASC at millimolar concentrations (1-100 mM) is selectively toxic to a variety of cultured tumor cell lines, including solid tumors (e.g. melanoma, ovarian, prostate, pancreatic, colon), as well as hematological malignancies (lymphoma, leukemia) [25, 26]. In animal studies, parenteral L-ASC given daily at high doses (4-6,5 g/kg body weight) revealed no body weight loss, histopathological changes nor abnormal rates of mortality. Importantly, parenteral L-ASC administration resulted in millimolar plasma concentrations and had antitumor activity in several xenograft [25], and syngeneic murine models, e.g. murine hepatoma [26], and pancreatic cancer [25]. Importantly, although B cell lymphomas were among the most sensitive cell lines for L-ASC *in vitro,* no significant benefit was reported in mice [4].

### Combinations of L-ASC with other antitumor agents

Variable data exist on the interaction of L-ASC with anti-cancer chemotherapeutics, revealing both synergistic and antagonistic interactions. Studies using human breast cancer cell line MCF7 reported that ASC enhanced the antitumor activity of doxorubicin, cisplatin, and etoposide [26]. High-dose (4g/kg) of i.p. L-ASC improved gemcitabine antitumor effect in a mouse model of pancreatic cancer [27]. L-ASC was also reported to improve ROS-generating therapeutic modalities such as radiotherapy and photodynamic therapy [28]. Additionally, in a mouse models of ovarian cancer, L-ASC combined with the conventional chemotherapeutics carboplatin and paclitaxel, synergistically inhibited tumor growth [29]. On the other hand, treatment of leukemia and lymphoma cell lines with DHA impaired cytotoxic effects of various chemotherapeutic agents including doxorubicin, cisplatin, methotrexate, both in cell cultures *in vitro* and *in vivo* in human lymphoma xenografts to mice [30]. Moreover, studies in multiple myeloma and lymphoma murine models revealed antagonistic interactions of L-ASC and bortezomib [31]. Taken together, the effects of ASC on anti-cancer therapies vary depending on cancer type, oxidation level and mode of administration.

### Mechanisms of L-ASC antitumor activity

The mechanism by which L-ASC exerts its antitumor effects has been widely investigated. The studies by Chen [3] paradoxically revealed pro-oxidant properties of L-ASC. High L-ASC concentrations in the presence of transition metal ions in serum proteins extracellularly generated H₂O₂, which was toxic to tumor cells. Other studies presented distinct mechanisms of L-ASC antitumor activity, involving L-ASC transport to cells and intracellular ROS generation. Hong et al. [32] have shown that the levels of SVCT-2, which transports reduced L-ASC to cells, determine chemosensitivity of breast cancer cells to L-ASC. Very recently Yun et al. [33] presented that L-ASC is selectively toxic to cancer cells with Mitogen-Activated Protein Kinases (MAPK) pathway activating mutations (B-Raf, K-Ras), which lead to overexpression of GLUT-1, a transporter utilized by cancer cells to uptake an oxidized form of L-ASC.

### L-ASC in treating patients with cancer

Treatment of cancer with high-dose L-ASC has a long and controversial history. In the middle of 20^{th} century several case reports presented beneficial effects of high dose (10 g daily) intravenous L-ASC administration in cancer patients. Studies by Nobel Prize winner L. Pauling [34] conducted on a group of patients with advanced cancers revealed over 4-fold increased mean survival time (MST) for L-ASC-treated subjects. These results implied that L-ASC may be beneficial in the treatment of at least some types of advanced cancer and encouraged further clinical trials. However, the following randomized controlled clinical trials [35] reported no benefit of oral L-ASC (10 g daily) treatment. Nonetheless, later pharmacokinetic studies reported substantial differences in L-ASC plasma concentrations depending on the route of delivery, from maximally 300 µM for oral administration, up to 20 mM for intravenous (i.v.) delivery. These results triggered further clinical trials with L-ASC given i.v. in high (10-100 g) doses daily or in other delivery schemes. So far, there is no convincing evidence from high-quality, randomized control clinical trials that L-ASC itself has robust antitumor effects. However, initial reports from clinical trials are promising. Results of phase I trials show that high-dose i.v. ASC mitigates conventional therapy-associated side effects and do not interfere with chemotherapy in breast [36], pancreatic [37] and ovarian cancer patients [29]. In majority of reports, parenteral and oral L-ASC is well tolerated with exception of patients with renal dysfunction and deficiency of glucose-6-phosphate dehydrogenase. Clinical trials evaluating the efficacy of parenteral high-dose L-ASC in combination with conventional therapy in lung, prostate, pancreatic, ovarian, and other cancers are ongoing. Although high-dose i.v. L-ASC administration has a chance to be beneficial for cancer patients, this route of administration is inconvenient and little cost-effective.

### Combinations of L-ASC with arsenic trioxide in hematological malignancies

Arsenic trioxide was demonstrated to inhibit TXNR in breast cancer cells [11], however its mechanisms of activity are much more complex, involving PML-RAR alpha degradation in acute promyelocytic leukemia [38], or inhibition of histone deacetylase 6 activity [39]. Importantly, numerous studies reported the beneficial effects of L-ASC on arsenic trioxide cytotoxicity. Dai et al. [40] reported that L-ASC sensitizes acute myeloid leukemia and B cell lymphoma cell lines to arsenic trioxide and that the combination of these compounds increased the survival of mice bearing P388D lymphoma. Later, the preclinical efficacy of this combination was also confirmed in other neoplasms, e.g. multiple myeloma [41] and ovarian cancer [42]. In clinical trials, the treatment combining arsenic trioxide, L-ASC and melphalan (MAC regimen) was effective and well tolerated in refractory/relapsed multiple myeloma patients (48% objective response) [43]. However, the clinical efficacy of arsenic trioxide + L-ASC was limited in patients with advanced lymphoid malignancies [44] and refractory acute myeloid leukemia [45].

Despite a significant number of available or investigated treatments for B-cell malignancies, there still exists a need for novel therapies directed specifically to the tumor cells, whose detrimental effect on healthy cells would be limited. Moreover, most of the available treatments involve non-specific, myelotoxic chemotherapy and require i.v. administration, which potentiates the patient's discomfort during the treatment. Therefore, novel therapies, which would rely on orally-administered, selective medications are highly desirable.

### DISCLOSURE OF INVENTION

The scope of the present invention is defined by the appended claims. Any other disclosure in the present description, regardless of whether it is indicated as preferred or examplified, does not form part of the present invention.

The inventors of the present invention have demonstrated synergistic interaction between thioredoxin reductase (TXNR) inhibitors, in particular AUR, and ascorbate (ASC). This synergistic effect between TXNR inhibitors and ASC manifested by potent cytotoxic effects of this combination to human BL cell line Raji. Importantly, the concentrations of L-ASC, which exert synergistic effects, are in the range between 100 - 200 µM, and are achievable after oral administration. Moreover, a similar synergistic effect was observed for D-ASC, an enantiomer of L-ASC with similar redox properties, but not for L-DHA, an oxidized form of L-ASC, which is intracellularly converted to L-ASC. Additionally, the combined SK053 and L-ASC treatment triggered massive production of ROS in Raji cells. The ROS decreased to control levels in the presence of catalase, an enzyme which scavenges H₂O₂, suggesting that H₂O₂ is the dominant ROS produced by the combination treatment. Importantly, the inventors did not observe synergistic cytotoxic effects of the combination to normal B cells isolated from human tonsils.

Therefore, the present invention provides a combination of a thioredoxin reductase (TXNR) inhibitor - auranofin - and ascorbate (ASC) for use in treatment of B cell malignancies. Preferably, auranofin is used as the TXNR inhibitor in the combination of the invention. Also in the preferred embodiment the ASC is selected from a group comprising L-ascorbate (L-ASC), D-ascorbate (D-ASC), or a combination thereof. Most preferably, sodium L-ascorbate or sodium D-ascorbate is used in the combination of the invention. However, other mineral salts of ascorbic acid, such as calcium and potassium salts are also contemplated as a part of the combination of the invention.

The combination of the invention is preferably used in the treatment of the B cell malignancies selected from a group comprising non Hodgkin lymphoma, in particular a diffuse large B cell lymphoma, mantle cell lymphoma and Burkitt lymphoma.

According to the invention the TXNR inhibitor and ASC can be administered simultaneously, separately or sequentially. Preferably, both the TXNR inhibitor and ASC are administered orally. Most preferably, the TXNR inhibitor and ASC in the combination of the invention are formulated into a single dosage form.

Auranofin, as well as the ASC can be formulated into the suitable dosage form by using standard methods and standard auxiliary agents known in the art. Preferably, AUR and ASC can be used in the dosage form currently available on the market.

The present invention also provides a method of treatment of B cell malignancy, such as diffuse large B cell lymphoma, mantel cell lymphoma and Burkitt lymphoma, by administrating TXNR inhibitor in combination of ASC. In the method of treatment, a combination of AUR and either L-ASC or D-ASC are used. The TXNR inhibitor and ASC can be administered simultaneously, separately or sequentially. In the preferred embodiment at least one of the TXNR inhibitor and ASC is administered orally. In the most preferred embodiment both the TXNR inhibitor and ASC are administered orally.

A very important effect of the present invention is that the synergistic cytotoxic effects between auranofin (AUR), and L- and D-ASC, is demonstrated toward malignant but not to normal B cells. Several other advantages concerning the present invention are listed below.

L-ASC is a dietary supplement and AUR an anti-rheumatic agent used already for decades. Considering a known pharmacokinetics and safety profile of both compounds, their combination could pursue quickly into clinical trials provided that animal studies confirmed the effects observed in cell culture.

In addition, the synergistic effects occur for the L-ASC concentrations as low as 100 - 200 µM, which are easily achievable after oral administration. Currently in clinics two modes of L-ASC administration are used: i.v. and oral. Intravenous protocol involves the slow infusion of high doses of L-ASC. This infusion of a bolus results in a peak plasma concentrations over 10 mM, followed by a decrease to physiological levels within hours. Administration of high oral doses of L-ASC leads to approximately 220 µM, while the liposomal formulation of L-ASC to as high as 400 µM sustainable plasma concentration. The fact that only 100 - 200 µM L-ASC enhances AUR cytotoxic effects shows that the combination of the invention may be used as an effective oral formulation. This simplifies drug administration, lowers the costs of the therapy and eliminates inconvenience associated with intravenous administration of L-ASC.

Moreover, the efficacy of the TXNR inhibitors in combination with ASC was tested in several tumor cell lines. It was demonstrated that the B cell-derived neoplasms are particularly sensitive to the combination of the invention. B cell receptor (BCR) signaling has a central role in B cell development. Normal B cells are selected for medium level of BCR signaling. Lack of BCR signaling leads to apoptosis, but also hyper-activation of BCR, like self-recognition during negative selection, triggers cell death. The activation status of mitogen activated kinases, which are activated downstream to BCR, is controlled by ROS-sensitive phosphatases [46]. The increased phosphorylation of BCR-downstream kinases, AKT and ERK1/2 in Raji cells subjected to SK053 has been observed. Importantly, pretreatment with ERK1/2 phosphorylation inhibitor, U0126, attenuated SK053-induced apoptosis, suggesting that ERK1/2 signaling contributes to cell death [47]. Altogether, without limiting to any theory, it is presumed that H₂O₂ generated by the combination of TXNR inhibitors and L-ASC leads to BCR hyper-activation and triggers cells death.

A combination of L-ASC and another TXNR inhibitor, arsenic trioxide, is already clinically exploited, and provides clinical benefit in selected types of tumors (see Background section). Considering cardiotoxicity of arsenic trioxide, and its antitumor activity mainly restricted to acute promyelocytic leukemia, the use of another TXNR inhibitor, AUR, in combination with L-ASC is superior.

AUR is tested in clinical trials as a monotherapy in B-CLL (6 mg/ day, oral), however only transient effects are observed in treated patients due to compensatory response. Therefore, dose escalation studies are underway. The addition of L-ASC could boost AUR activity and improve treatment efficacy in CLL patients.

L-ASC potentiates TXNR inhibitors cytotoxic effects via generation of H₂O₂. D-ASC is as active as L-ASC, while oxidized form, L-DHA, is not effective. It strongly suggest that extracellularly generated H₂O₂ accounts for cytotoxicity of the combination treatment. Considering that ASC transport to cells and its metabolism are at least partially stereo-selective, the alternative use of D-ASC may reduce unwanted effects in normal cells. However, as the absorption of D-ASC after oral administration is limited, D-ASC isomer should rather be delivered parenterally. Therefore, in an alternative embodiment when D-ASC is used in the combination of the invention, the ASC is administered parenterally.

### BRIEF DESCRIPTION OF DRAWINGS

The subject of the invention was illustrated in a drawing, in which:
Figure 1 illustrates cytostatic/cytotoxic effects of sodium ascorbate in combination with SK053 (A) and AUR (B) assessed with MTT assay.
Figure 2 shows a fraction of non-viable cells upon treatment with L-ASC in combination with SK053 (A) and AUR (B). The number of non-viable cells was determined by flow cytometry, as a fraction of propidium iodide-positive cells.
Figure 3 illustrates cytotoxic effects of ascorbate stereoisomers L-ASC, D-ASC and an oxidized form of L-ASC (L-DHA).
Figure 4 illustrates intracellular ROS levels in Raji cells incubated with L-ASC and SK053, separately and in combination. (A) ROS levels were evaluated using CM-H2-DCFDA fluorescent probe, as described in the text. (B) Raji cells were subjected to L-ASC and SK053 treatment with and without the addition of 100 µg/ml catalase.
Figure 5 shows a fraction of non-viable cells upon treatment with L-ASC in combination with SK053 and AUR. The number of non-viable cells was determined as describes in Example 1.
Figure 6 illustrates cytotoxic effects of L-ASC in combination with AUR and SK053 to MCF7 breast cancer cells. The number of viable cells was determined by crystal violet assay.

### EXAMPLES

### Example 1. L-ASC in combination with TXNR inhibitors synergistically kill malignant B-lymphocytes

Raji cells were grown in RPMI medium supplemented with 10% fetal bovine serum and antibiotic/antimycotic solution. Raji cells were incubated for 15 min at 37 °C with 100 µM or 200 µM L-ASC and then TXNR inhibitors, SK053 or AUR, were added and the cells were cultured for additional 48 hours. Cytostatic/cytotoxic effects of treatment were assessed by:

### (a) MTT (3-[4,5-dimethylthiazol-2-yl]-2,5-diphenyltetrazolium bromide) assay

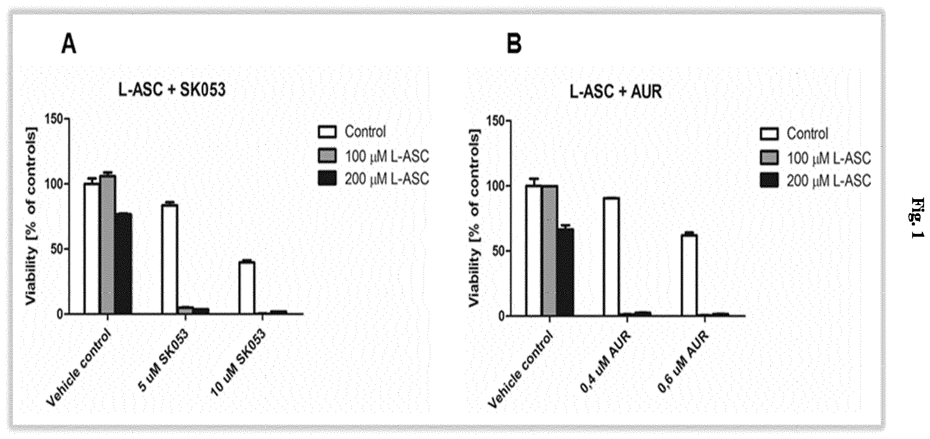

20 µl of MTT (5mg/ml in PBS) was added to 100 µl of cells and maintained in cell culture conditions. After 2 hours, 100 µl of solubilization buffer (10% SDS, 0,01N HCl) was added to each well and plates were maintained in a cell culture incubator overnight. The absorbance was measured at 560 nm using a microplate reader (ASYS UVM 340, Biochrom). The relative viability was calculated according to the following formula: % viability = [(Am - Ab)/(Ac - Ab)] × 100%, where Am is the measured absorbance, Ab is the background absorbance, and Ac is the absorbance of vehiculum-treated controls (DMSO). As illustrated in Fig. 1, L-ASC alone at 100 µM and 200 µM concentrations did not affect the viability of Raji cells. However, in both concentrations it remarkably increased the cytostatic/cytotoxic effects of SK053 and AUR. These results reveal strong synergistic interactions between L-ASC and TXNR inhibitors, SK053 and AUR.

### (b) Quantitation of the number of dead cells with propidium iodide staining using flow cytometry

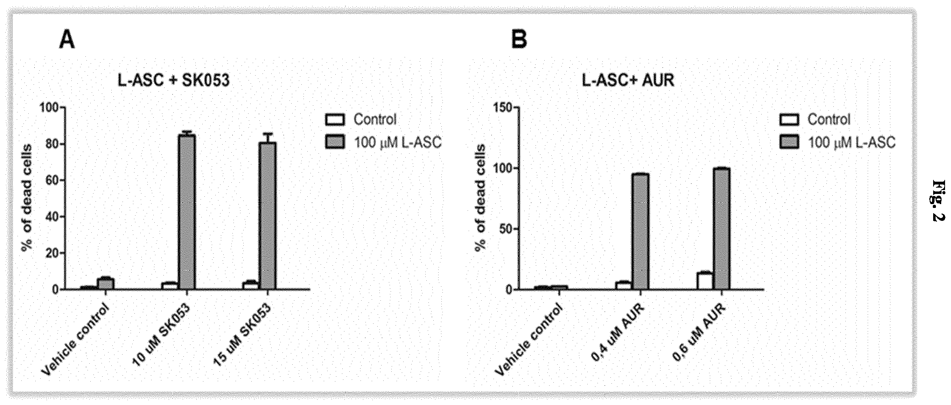

Raji cells were subjected to 100 µM L-ASC and TXNR inhibitors as described in section (a) above. 100 µl of cells were mixed with 100 µl of propidium iodide solution (4 µg/ml in PBS) and the number of propidium iodide-positive cells (dead cells) was assessed using flow cytometer Accuri C6. As depicted in Fig. 2, the treatment of Raji cells with TXNR inhibitors and with L-ASC alone did not trigger massive cell death (< 10% of propidium iodide-positive cells). In contrast, the treatment of Raji cells with the combination of TXNR inhibitors and L-ASC resulted in nearly 100% of dead cells. These results confirm that 100 µM L-ASC remarkably enhances the cytotoxic activity of TXNR inhibitors, SK053 and AUR.

### Example 2. L- or D-ASC, but not L-DHA, in combination with TXNR inhibitors, synergistically kill lymphoma cells

The efficacy of two ascorbate stereoisomers, L-ASC and D-ASC, as well as the oxidized form, L-DHA, were compared. As presented in Fig. 3, D-ASC showed a similar synergistic interaction with TXNR inhibitors as L-ASC. It suggests that the configuration on the 5^{th} carbon of ASC is not essential for the synergism with TXNR inhibitors. In contrast, the oxidized form of L-ASC, L-DHA, had no impact on cytotoxicity of SK053 nor AUR to Raji cells.

The above results suggest that only reduced, both L- and D-ASC isomers, but not the oxidized form, L-DHA, produce synergistic interaction with TXNR inhibitors. This finding is in agreement with previously observed mechanism of cytotoxic activity of millimolar concentrations of L-ASC alone to lymphoma cell line JLP-119 [3]. Moreover, as D-ASC is not efficiently transported to cells, we conclude that extracellularly localized ASC accounts for increased cytotoxicity of the combination treatment.

### Example 3. (Reference)

### The combination treatment triggers accumulation of H₂O₂ in Raji cells

The level of intracellular ROS in Raji cells treated with L-ASC and SK053 alone or in combination was evaluated. Raji cells were loaded with CM-H2-DCFDA fluorescent probe (Molecular Probes) according to the manufacturer's protocol, using 1 µM dye concentration for 30 min. at 37 °C. Next, 100 µM ASC and 20 µM SK053 were added to labeled cells, separately and in combinations, and cells were incubated at 37°C. After 8 hours cells were harvested in PBS and the intensity of green fluorescence was analyzed by flow cytometry (Accuri C6).

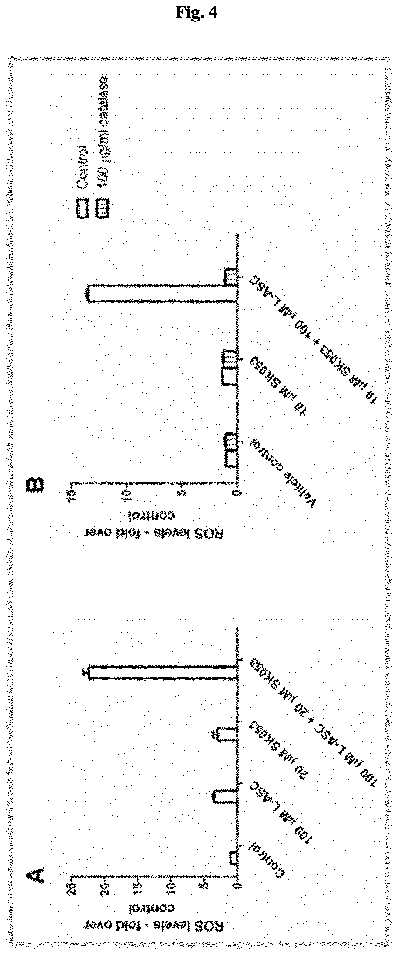

As illustrated in Fig. 4A, SK053 and L-ASC separately only slightly increased ROS levels, but in combination, they triggered potent ROS increase. Notably, ROS were at the control levels when Raji cells were incubated with SK053, L-ASC, and the combination of both compounds, in the presence of 100 µg/ml catalase, the H₂O₂ scavenging enzyme (Fig. 4B). These results show that the concomitant treatment of Raji cells with SK053 and L-ASC dramatically increases intracellular ROS levels. Presumably, the extracellularly generated H₂O₂ rapidly diffuses through membranes and triggers intracellular ROS increase.

### Example 4. No synergistic cytotoxic effects of the combination treatment are observed in normal B cells

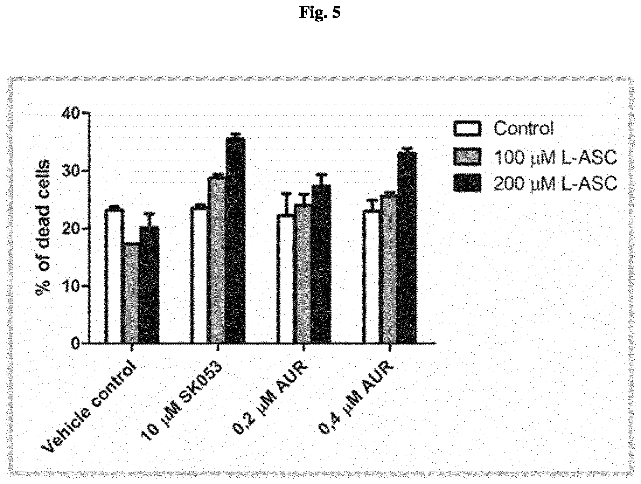

The effects of the combination of TXNR inhibitors with L-ASC to normal B cells isolated from human tonsils were assessed. Human tonsils were derived from subjects undergoing tonsillectomy. After resection, tonsils were cut into small pieces and incubated in the RPMI medium supplemented with collagenase and DNase for 30 min at 37 °C with gentle shaking and filtered through 70 µm cell strainer. After washing in PBS, mononuclear cells were isolated with Histopaque 1077 density gradient and subjected to CD19 negative selection using EasySep™ Human B Cell Enrichment Kit (STEMCELL Technologies). The purity of isolated B cells exceeded 95%. Subsequently, B cells were suspended in RPMI medium supplemented with 10% fetal bovine serum and antibiotic/antimycotic solution, and incubated with L-ASC, SK053, AUR, exactly as Raji cells (described in Example 1). After 48 hours of incubation, the number of non-viable cells was determined by flow cytometry, as the fraction of propidium iodide positive cells. As presented in Fig. 5, the synergistic cytotoxic effect between none of the TXNR inhibitors and L-ASC was observed. This experiment was repeated in three different healthy donors. In conclusion, the combination of TXNR inhibitors and L-ASC is selectively toxic to tumor cells, while sparing their normal counterparts.

### Example 5. Higher concentrations of L-ASC are needed to potentiate cytotoxic activity of TXNR inhibitors in breast cancer cell line MCF7

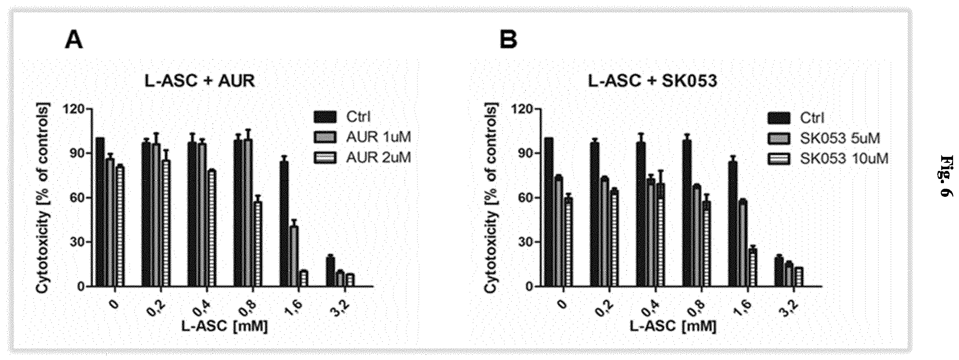

The effects of the combination of TXNR inhibitors with L-ASC to breast cancer cell line MCF7 was assessed. We applied the treatment scheme as in Raji cells (described in Example 1). After 48 hours of incubation, cell viability was determined by crystal violet cytotoxicity assay, according to protocol described in [48]. As presented in Fig. 6, only concentration above 1 mM L-ASC increased cytotoxicity of AUR and SK053. These results reveal that B cell tumors are particularly sensitive to the combination of low dose L-ASC with TXNR inhibitors.

### REFERENCES

1. Sandlund, JT, Burkitt lymphoma: staging and response evaluation. Br J Haematol, 2012. 156(6): p. 761-5.
2. Barr, PM, et al., Phase 2 study of imexon, a prooxidant molecule, in relapsed and refractory B-cell non-Hodgkin lymphoma. Blood, 2014. 124(8): p. 1259-65.
3. Chen, Q, et al., Pharmacologic ascorbic acid concentrations selectively kill cancer cells: action as a pro-drug to deliver hydrogen peroxide to tissues. Proc Natl Acad Sci U S A, 2005. 102(38): p. 13604-9.
4. Shatzer, AN, et al., Ascorbic acid kills Epstein-Barr virus positive Burkitt lymphoma cells and Epstein-Barr virus transformed B-cells in vitro, but not in vivo. Leuk Lymphoma, 2013. 54(5): p. 1069-78.
5. Irwin, ME, et al., Redox control of leukemia: from molecular mechanisms to therapeutic opportunities. Antioxid Redox Signal, 2013. 18(11): p. 1349-83.
6. Gromer, S, et al., The thioredoxin system--from science to clinic. Med Res Rev, 2004. 24(1): p. 40-89.
7. Selenius, M, et al., Effects of redox modulation by inhibition of thioredoxin reductase on radiosensitivity and gene expression. J Cell Mol Med, 2012. 16(7): p. 1593-605.
8. Smart, DK, et al., Thioredoxin reductase as a potential molecular target for anticancer agents that induce oxidative stress. Cancer Res, 2004. 64(18): p. 6716-24.
9. Gandin, V, et al., Metal- and Semimetal-Containing Inhibitors of Thioredoxin Reductase as Anticancer Agents. Molecules, 2015. 20(7): p. 12732-56.
10. Hashemy, SI, et al., Motexafin gadolinium, a tumor-selective drug targeting thioredoxin reductase and ribonucleotide reductase. J Biol Chem, 2006. 281(16): p. 10691-7.
11. Lu, J, et al., Targeting thioredoxin reductase is a basis for cancer therapy by arsenic trioxide. Proc Natl Acad Sci U S A, 2007. 104(30): p. 12288-93.
12. Chew, EH, et al., Thioredoxin reductase inhibition by antitumor quinols: a quinol pharmacophore effect correlating to antiproliferative activity. FASEB J, 2008. 22(6): p. 2072-83.
13. Fang, J, et al., Thioredoxin reductase is irreversibly modified by curcumin: a novel molecular mechanism for its anticancer activity. J Biol Chem, 2005. 280(26): p. 25284-90.
14. Klossowski, S, et al., Studies toward novel peptidomimetic inhibitors of thioredoxin-thioredoxin reductase system. J Med Chem, 2012. 55(1): p. 55-67.
15. Trzeciecka, A, et al., Dimeric peroxiredoxins are druggable targets in human Burkitt lymphoma. Oncotarget, 2015.
16. Kean, WF, et al., Auranofin. Br J Rheumatol, 1997. 36(5): p. 560-72.
17. Fan, C, et al., Enhancement of auranofin-induced lung cancer cell apoptosis by selenocystine, a natural inhibitor of TrxR1 in vitro and in vivo. Cell Death Dis, 2014. 5: p. e1191.
18. Pessetto, ZY, et al., Drug repurposing for gastrointestinal stromal tumor. Mol Cancer Ther, 2013. 12(7): p. 1299-309.
19. Liu, N, et al., Clinically used antirheumatic agent auranofin is a proteasomal deubiquitinase inhibitor and inhibits tumor growth. Oncotarget, 2014. 5(14): p. 5453-71.
20. Mirabelli, CK, et al., Evaluation of the in vivo antitumor activity and in vitro cytotoxic properties of auranofin, a coordinated gold compound, in murine tumor models. Cancer Res, 1985. 45(1): p. 32-9.
21. Fiskus, W, et al., Auranofin induces lethal oxidative and endoplasmic reticulum stress and exerts potent preclinical activity against chronic lymphocytic leukemia. Cancer Res, 2014. 74(9): p. 2520-32.
22. Celegato, M, et al., Preclinical activity of the repurposed drug auranofin in classical Hodgkin lymphoma. Blood, 2015. 126(11): p. 1394-7.
23. Kiebala, M, et al., Dual targeting of the thioredoxin and glutathione antioxidant systems in malignant B cells: a novel synergistic therapeutic approach. Exp Hematol, 2015. 43(2): p. 89-99.
24. Banhegyi, G, et al., Subcellular compartmentation of ascorbate and its variation in disease states. Biochim Biophys Acta, 2014. 1843(9): p. 1909-16.
25. Chen, Q, et al., Pharmacologic doses of ascorbate act as a prooxidant and decrease growth of aggressive tumor xenografts in mice. Proc Natl Acad Sci U S A, 2008. 105(32): p. 11105-9.
26. Verrax, J, et al., Pharmacologic concentrations of ascorbate are achieved by parenteral administration and exhibit antitumoral effects. Free Radic Biol Med, 2009. 47(1): p. 32-40.
27. Espey, MG, et al., Pharmacologic ascorbate synergizes with gemcitabine in preclinical models of pancreatic cancer. Free Radic Biol Med, 2011. 50(11): p. 1610-9.
28. Wei, Y, et al., Enhancement of photodynamic antitumor effect with pro-oxidant ascorbate. Lasers Surg Med, 2012. 44(1): p. 69-75.
29. Ma, Y, et al., High-dose parenteral ascorbate enhanced chemosensitivity of ovarian cancer and reduced toxicity of chemotherapy. Sci Transl Med, 2014. 6(222): p. 222ra18.
30. Heaney, ML, et al., Vitamin C antagonizes the cytotoxic effects of antineoplastic drugs. Cancer Res, 2008. 68(19): p. 8031-8.
31. Perrone, G, et al., Ascorbic acid inhibits antitumor activity of bortezomib in vivo. Leukemia, 2009. 23(9): p. 1679-86.
32. Hong, SW, et al., SVCT-2 in breast cancer acts as an indicator for L-ascorbate treatment. Oncogene, 2013. 32(12): p. 1508-17.
33. Yun, J, et al., Vitamin C selectively kills KRAS and BRAF mutant colorectal cancer cells by targeting GAPDH. Science, 2015.
34. Cameron, E, et al., Supplemental ascorbate in the supportive treatment of cancer: Prolongation of survival times in terminal human cancer. Proc Natl Acad Sci U S A, 1976. 73(10): p. 3685-9.
35. Moertel, CG, et al., High-dose vitamin C versus placebo in the treatment of patients with advanced cancer who have had no prior chemotherapy. A randomized double-blind comparison. N Engl J Med, 1985. 312(3): p. 137-41.
36. Vollbracht, C, et al., Intravenous vitamin C administration improves quality of life in breast cancer patients during chemo-/radiotherapy and aftercare: results of a retrospective, multicentre, epidemiological cohort study in Germany. In Vivo, 2011. 25(6): p. 983-90.
37. Monti, DA, et al., Phase I evaluation of intravenous ascorbic acid in combination with gemcitabine and erlotinib in patients with metastatic pancreatic cancer. PLoS One, 2012. 7(1): p. e29794.
38. Lallemand-Breitenbach, V, et al., Arsenic degrades PML or PML-RARalpha through a SUMO-triggered RNF4/ubiquitin-mediated pathway. Nat Cell Biol, 2008. 10(5): p. 547-55.
39. Qu, X, et al., Arsenic trioxide exerts antimyeloma effects by inhibiting activity in the cytoplasmic substrates of histone deacetylase 6. PLoS One, 2012. 7(2): p. e32215.
40. Dai, J, et al., Malignant cells can be sensitized to undergo growth inhibition and apoptosis by arsenic trioxide through modulation of the glutathione redox system. Blood, 1999. 93(1): p. 268-77.
41. Grad, JM, et al., Ascorbic acid enhances arsenic trioxide-induced cytotoxicity in multiple myeloma cells. Blood, 2001. 98(3): p. 805-13.
42. Ong, PS, et al., Differential augmentative effects of buthionine sulfoximine and ascorbic acid in As2O3-induced ovarian cancer cell death: oxidative stress-independent and -dependent cytotoxic potentiation. Int J Oncol, 2011. 38(6): p. 1731-9.
43. Berenson, JR, et al., Efficacy and safety of melphalan, arsenic trioxide and ascorbic acid combination therapy in patients with relapsed or refractory multiple myeloma: a prospective, multicentre, phase II, single-arm study. Br J Haematol, 2006. 135(2): p. 174-83.
44. Chang, JE, et al., Phase II study of arsenic trioxide and ascorbic acid for relapsed or refractory lymphoid malignancies: a Wisconsin Oncology Network study. Hematol Oncol, 2009. 27(1): p. 11-6.
45. Aldoss, I, et al., Adding ascorbic acid to arsenic trioxide produces limited benefit in patients with acute myeloid leukemia excluding acute promyelocytic leukemia. Ann Hematol, 2014. 93(11): p. 1839-43.
46. Turner-Ivey, B, et al., Role for Prdx1 as a specific sensor in redox-regulated senescence in breast cancer. Oncogene, 2013. 32(45): p. 5302-14.
47. Trzeciecka, A, et al., Dimeric peroxiredoxins are druggable targets in human Burkitt lymphoma. Oncotarget, 2015(accepted for publication Nov 14, 2015).
48. Firczuk, M, et al., Mutational analysis of peptidoglycan amidase MepA. Biochemistry, 2007. 46(1): p. 120-8.

## Claims

1. A combination of a thioredoxin reductase inhibitor, auranofin, and ascorbate for use in treatment of B cell malignancies.

2. The combination for use of claim 1, wherein ascorbate is selected from a group comprising L-ascorbate, D-ascorbate, or a combination thereof.

3. The combination for use of claim 1 or 2, wherein L-ascorbate or D-ascorbate is in the form of sodium L-ascorbate or sodium D-ascorbate, respectively.

4. The combination for use of anyone of claims 1-3, wherein the B-cell malignancy is selected from a group comprising non Hodgkin lymphoma: diffuse large B cell lymphoma, Burkitt lymphoma and mantle cell lymphoma.

5. The combination for use of anyone of claims 1-4, wherein the thioredoxin reductase inhibitor and ascorbate are to be administered simultaneously, separately or sequentially.

6. The combination for use of anyone of claims 1-5, wherein ascorbate is to be administrated orally.

7. The combination for use of anyone of claims 1-6, wherein both the thioredoxin reductase inhibitor and ascorbate are to be administered orally.

8. The combination for use of anyone of claim 1-7, wherein it is in a form of a single dosage form.

## Patentansprüche

1. Kombination von einem Thioredoxin-Reduktase-Inhibitor, Auranofin, und einem Ascorbat zur Verwendung in der Behandlung von B-Zell-Malignitäten.

2. Die Kombination zur Verwendung nach Anspruch 1, wobei das Ascorbat ausgewählt ist aus einer Gruppe umfassend L-Ascorbat, D-Ascorbat oder eine Kombination davon.

3. Die Kombination zur Verwendung nach Anspruch 1 oder 2, wobei L-Ascorbat oder D-Ascorbat in Form von Natrium-L-Ascorbat beziehungsweise Natrium-D-Ascorbat vorliegt.

4. Die Kombination zur Verwendung nach einem der Ansprüche 1 bis 3, wobei die B-Zell-Malignität ausgewählt ist aus einer Gruppe umfassend Non-Hodgkin-Lymphom: diffuses großzelliges B-Zell-Lymphom, Burkitt-Lymphom und Mantelzell-Lymphom.

5. Die Kombination zur Verwendung nach einem der Ansprüche 1 bis 4, wobei der Thioredoxin-Reduktase-Inhibitor und das Ascorbat gleichzeitig, getrennt oder nacheinander verabreicht werden.

6. Die Kombination zur Verwendung nach einem der Ansprüche 1-5, wobei das Ascorbat oral zu verabreichen ist.

7. Die Kombination zur Verwendung nach einem der Ansprüche 1-6, wobei sowohl der Thioredoxin-Reduktase-Inhibitor als auch Ascorbat oral zu verabreichen sind.

8. Die Kombination zur Verwendung nach einem der Ansprüche 1-7, wobei sie in Form einer Einzeldosierungsform vorliegt.

## Revendications

1. Combinaison d'un inhibiteur de la thiorédoxine-réductase, de l'auranofine, et d'un ascorbate à utiliser dans le traitement des malignités des cellules B.

2. La combinaison à utiliser selon la revendication 1, dans laquelle l'ascorbate est choisi dans un groupe comprenant le L-ascorbate, le D-ascorbate ou une combinaison de ceux-ci.

3. La combinaison à utiliser selon la revendication 1 ou 2, dans laquelle le L-ascorbate ou le D-ascorbate est sous la forme de L-ascorbate de sodium ou de D-ascorbate de sodium, respectivement.

4. La combinaison à utiliser selon l'une quelconque des revendications 1 à 3, dans laquelle la malignité des cellules B est choisie dans un groupe comprenant un lymphome non hodgkinien: lymphome diffus à grandes cellules B, lymphome de Burkitt et lymphome à cellules du manteau.

5. La combinaison à utiliser selon l'une quelconque des revendications 1 à 4, dans laquelle l'inhibiteur de thiorédoxine-réductase et l'ascorbate doivent être administrés simultanément, séparément ou séquentiellement.

6. La combinaison à utiliser selon l'une quelconque des revendications 1 à 5, dans laquelle l'ascorbate doit être administré par voie orale.

7. La combinaison à utiliser selon l'une quelconque des revendications 1 à 6, dans laquelle à la fois l'inhibiteur de thiorédoxine-réductase et l'ascorbate doivent être administrés par voie orale.

8. La combinaison à utiliser selon l'une quelconque des revendications 1 à 7, dans laquelle elle se présente sous la forme d'une forme de dosage unique.
